# EUROPEAN PATENT SPECIFICATION

(11) **EP 2 194 929 B1**
(45) Date of publication and mention of the grant of the patent: **03.10.2012**
(21) Application number: 08830887.9
(22) Date of filing: 15.08.2008
(51) Int. Cl.: A61F 2/44, A61F 2/00, A61F 2/02

(54) **SPINAL INTERBODY REPLACEMENT DEVICES**
VORRICHTUNGEN ZUM ERSATZ VON WIRBELZWISCHENKÖRPERN
DISPOSITIFS DE REMPLACEMENT INTERCORPORELS SPINAUX

(30) Priority: 27.08.2007 US 895730
(43) Date of publication of application: 16.06.2010
(73) Proprietor: Warsaw Orthopedic, Inc., Warsaw, IN 46581 (US)
(72) Inventor: FOLEY, Kevin Thomas, Germantown Tennessee 38139 (US); SHIPP, Kenneth S., Collierville Tennessee 38017 (US); CHEN, Richard Evan, Memphis Tennessee 38103 (US)
(74) Representative: Viering, Jentschura & Partner
(86) International application number: PCT/US2008/073331
(87) International publication number: WO 2009/035817

(56) References cited:
- WO-A-2004/093751
- US-A1- 2005 125 029
- US-A1- 2005 143 822
- US-A1- 2006 116 770

## Description

### BACKGROUND

The repair and reconstruction of bony structures is sometimes accomplished by fixing adjacent bony structures relative to one another, such as by a plate. In other instances, bone growth inducing material can be introduced between the adjacent bony structures, which over time results in a solid bony connection. In some instances, the adjacent bony structures are not sufficiently strong to maintain their patency as the bone heals or the bone grows between the adjacent structures through the bone growth inducing material. In these instances, mesh structures or cages have been provided to engage the adjacent bony structures to provide additional stability. The cages are generally hollow and can be configured to contact the harder cortical bone of the adjacent bony structures. The hollow portion of the cages can be filled with bone growth inducing material.

Devices have been positioned in a disc space between adjacent vertebrae to provide support while the adjacent vertebrae are fused to one another. Devices have also been provided to replace a removed vertebral body and to provide a support structure between the remaining vertebrae on either side of the one or more removed vertebral bodies. However, there remains a need for improved devices for replacing one or more vertebral bodies and/or to provide support of adjacent vertebrae with one or more devices in a disc space between adjacent vertebrae. The present invention is directed to satisfying these needs, among others.

US 2005/0125029 A1 discloses an implant designed to be inserted in the disk space defined between two adjacent vertebrae, the implant being in the form of a cage having a top transverse face with a convex profile and a bottom transverse face that is substantially straight.

US 2006/0116770 A1 discloses a vertebral replacement device for supporting adjacent vertebrae, and US 2005/0143822 A1 discloses an intervertebral fusion cage.

### SUMMARY

The present invention provides a spinal interbody fusion member according to claim 1. Further embodiments of the spinal interbody fusion member of the present invention are described in the dependent claims.

There are provided devices for replacement of one or more vertebral bodies to support the remaining vertebrae. Also provided are one or more devices that support adjacent vertebrae in the disc space between the adjacent vertebrae.

According to one aspect, a spinal interbody fusion member comprises a body including an anterior side, a posterior side, and a pair of opposite sides extending between the anterior and posterior sides. The body also defines a superior bearing surface and an opposite inferior bearing surface. The superior bearing surface includes a convexly curved profile between the anterior and posterior sides and the inferior bearing surface includes a planar profile between the anterior and posterior sides.

According to another aspect, a spinal interbody fusion member comprises a body including an anterior wall, a posterior wall, and a pair of opposite sidewalls extending between the anterior and posterior walls. The walls define a superior bearing surface and an opposite inferior bearing surface and a chamber opening through the superior and inferior bearing surfaces. The superior bearing surface includes a convexly curved profile between the anterior and posterior walls and the inferior bearing surface includes a planar profile between the anterior and posterior walls. The inferior and superior bearing surfaces taper toward a leading end nose at a posterior side of the posterior wall.

According to another aspect, a vertebral body replacement device comprises a connecting member including a body extending along a longitudinal axis between a superior end and an opposite inferior end. The connecting member includes an upper extension extending from the superior end and a lower extension extending from the inferior end. The superior end further includes at least one planar surface section extending about the upper extension and the inferior end includes a concavely curved surface extending about the lower extension. The replacement device further includes upper and lower disc replacement members removably attached to the connecting body about respective ones of the upper and lower extensions. The upper disc replacement member includes a superior bearing surface for contacting an endplate of an adjacent vertebra and an opposite inferior bearing surface including at least one planar profile positioned in abutting engagement with the at least one planar surface section of the superior end of the connecting body. The lower disc replacement member includes an inferior bearing surface for contacting an endplate of an adjacent vertebra and an opposite superior bearing surface including a convexly curved profile positioned in abutting engagement with the concavely curved surface of the inferior end of the connecting body.

According to a further aspect, a vertebral body replacement device comprises a connecting member including a body extending along a longitudinal axis between a superior end and an opposite inferior end. The connecting member includes an upper extension extending from the superior end and a lower extension extending from the inferior end. The vertebral replacement device further comprises upper and lower disc replacement members removably attached to the connecting body about respective ones of the upper and lower extensions. The upper disc replacement member includes a superior bearing surface for contacting an endplate of an upper vertebra with the superior bearing surface including a convexly curved profile between anterior and posterior sides of the upper disc replacement member. The lower disc replacement member includes an inferior bearing surface for contacting an endplate of a lower vertebra with the inferior bearing surface including at least one planar profile extending over a substantial portion of the inferior bearing surface from an anterior side of the lower disc replacement member toward a posterior side of the lower disc replacement member.

These and other aspects will be discussed further below.

### DESCRIPTION OF THE FIGURES

Fig. 1A is a diagrammatic view of a spinal column segment and a vertebral replacement device positioned between vertebrae to replace one or more removed vertebrae.
Fig. 1B is a diagrammatic view of a spinal column segment and a disc replacement member in a disc space between adjacent vertebrae.
Fig. 2 is a perspective view of a disc replacement member of the vertebral replacement device of Fig. 1B.
Fig. 3 is a plan view of the disc replacement member of Fig. 2.
Fig. 4 is a left end elevation view of the disc replacement member of Fig. 3.
Fig. 5 is a side elevation view of the disc replacement member of Fig. 2.
Fig. 6 is an enlarged view of the portion of the disc replacement member indicated in area VI of Fig. 5.
Fig. 7 is an enlarged view of the portion of the disc replacement member indicated in area VII of Fig. 5.
Fig. 8 is a plan view of the disc replacement member of Fig. 2 looking at the side opposite that of Fig. 3.
Fig. 9 is a section view through line IX-IX of Fig. 8.
Fig. 10 is a section view through line X-X of Fig. 8.
Fig. 11 is an elevation view of a connecting member of the vertebral replacement device of Fig. 1A.
Fig. 12 is an elevation view of the connecting member of Fig. 11 looking at the side of the connecting member opposite the side shown in Fig. 11.
Fig. 13 is a section view along line XIII-XIII of Fig. 12.
Fig. 14 is an enlarged view of the portion of the connecting member indicated in area XIV of Fig. 13.
Fig. 15 is an enlarged view of the portion of the connecting member indicated in area XV of Fig. 13.
Fig. 16 is a perspective view looking toward an end of the connecting member of Fig. 11.
Fig. 17 is a section view along line XVII-XVII of Fig. 16.

### DESCRIPTION OF THE ILLUSTRATED EMBODIMENTS

For the purpose of promoting an understanding of the principles of the invention, reference will now be made to the illustrated embodiments thereof and specific language will be used to describe the same. It will nevertheless be understood that no limitation of the scope of the invention is thereby intended. Any such alterations and further modifications in the invention, and any such further applications of the principles of the invention as described herein are contemplated as would normally occur to one skilled in the art to which the invention relates.

The disclosed embodiments relate to devices for replacing one or more vertebral bodies in the spinal column and/or one or more disc spaces between adjacent vertebrae. It is contemplated that the replacement devices will support vertebrae during fusion thereof. It is further contemplated that one or more components of the vertebral replacement devices are positionable in a disc space between adjacent vertebrae for supporting the adjacent vertebrae during fusion thereof. Applications in non-fusion procedures are also contemplated

The device is configured to engage the endplates of the vertebrae at opposite ends of the device. In one embodiment, the device has a tubular form with a hollow chamber extending therethrough and opening at each end. The vertebrae are supported by the device while the chamber contains bone growth inducing or osteogenetic material. The ends of the device include engaging members that engage the adjacent vertebral endplate. In a further embodiment, one of the ends of the device includes a convexly curved configuration while the other of the ends of the device includes a planar configuration.

In one embodiment, the device is a vertebral replacement device that includes a connecting member and an upper member attached to an upper end of the connecting member and a lower member attached to a lower end of the connecting member. The upper and lower members each have opposite end surfaces. The end surfaces of a respective upper or lower member have different configurations from one another. In one embodiment, one of the end surfaces has a convexly curved configuration and the opposite end surface has a planar configuration. The ends of the connecting member also include different configurations to accept respective ones of the curved and planar end surface configurations of the adjacent upper and lower member attached thereto.

Each of the upper and lower members and the connecting member has the same cross-sectional shape in the plane transverse to the central axis of the assembled device. In one embodiment, the shape is defined by a convexly curved anterior wall, a linear posterior wall, and opposite sidewalls that converge toward one another toward the posterior wall. Other cross-sectional shapes are also contemplated, including circular, racetrack-shaped, rectangular, square, oval, D-shaped, triangular, boomerang, kidney, or other polygonal shape. Each of the upper and lower members includes an interior chamber surrounded by the walls thereof. The connecting member can also include an interior chamber that generally aligns with the interior chambers of the upper and lower members when engaged thereto. Other embodiments contemplate one or more sides of the upper member, lower member or connecting member are open into the chamber.

In one embodiment, the upper and lower members are telescopically and non-rotatably engaged with the respective end of the connecting member. The connecting member includes an upper extension and a lower extension extending therefrom. The upper and lower extensions are in the form of substantially continuous rings extending around the respective ends of the connecting member. The upper and lower members are positioned about the respective adjacent extension and axially secured thereto with an engaging member extending between the extension and the upper or lower member positioned thereabout. In another embodiment, extensions are provided on the upper and lower members, and these extensions are received in an interior chamber or opening at respective ends of the connecting member. Each of the upper and lower extensions, and each of the chambers of the upper and lower members, has a non-circular cross-section and interface to prevent relative rotation between the connecting member and the upper or lower member positioned about the extension. In one embodiment, the upper and lower extensions of the connecting member each include an engaging member which fits into a receptacle in the inner wall surface of the respective upper and lower members to axially secure the respective upper and lower members to the connecting member.

Any one or all of the components of the vertebral replacement devices can be made from any biocompatible material, including synthetic or natural autograft, allograft or xenograft tissues, and can be resorbable or non-resorbable in nature. Examples of tissue materials include hard tissues, connective tissues, demineralized bone matrix and combinations thereof. Further examples of resorbable materials are polylactide, polyglycolide, tyrosine-derived polycarbonate, polyanhydride, polyorthoester, polyphosphazene, calcium phosphate, hydroxyapatite, bioactive glass, and combinations thereof. Further examples of non-resorbable materials are non-reinforced polymers, carbon-reinforced polymer composites, PEEK and PEEK composites, shape-memory alloys, titanium, titanium alloys, cobalt chrome alloys, stainless steel, ceramics and combinations thereof and others as well.

Any suitable osteogenetic material or composition is contemplated for placement within the chambers defined by the components or the vertebral replacement device. Such osteogenic material includes, for example, autograft, allograft, xenograft, demineralized bone, synthetic and natural bone graft substitutes, such as bioceramics and polymers, and osteoinductive factors. Where bony material is placed within the chambers of the components of the vertebral replacement device, the material can be pre-packed into the hollow chambers before the device is implanted, or can be pushed through the plurality of wall openings after the device is in position in the spinal column. A separate carrier to hold the materials within the chambers of the device can also be used. These carriers can include collagen-based carriers, bioceramic materials, such as BIOGLASS®, hydroxyapatite and calcium phosphate compositions. The carrier material can be provided in the form of a sponge, a block, folded sheet, putty, paste, graft material or other suitable form. Moreover, the osteogenetic compositions contained within the vertebral replacement device can comprise an effective amount of a bone morphogenetic protein, transforming growth factor β1, insulin-like growth factor 1, platelet-derived growth factor, fibroblast growth factor, LIM mineralization protein (LMP), and combinations thereof or other therapeutic or infection resistant agent, held within a suitable carrier material.

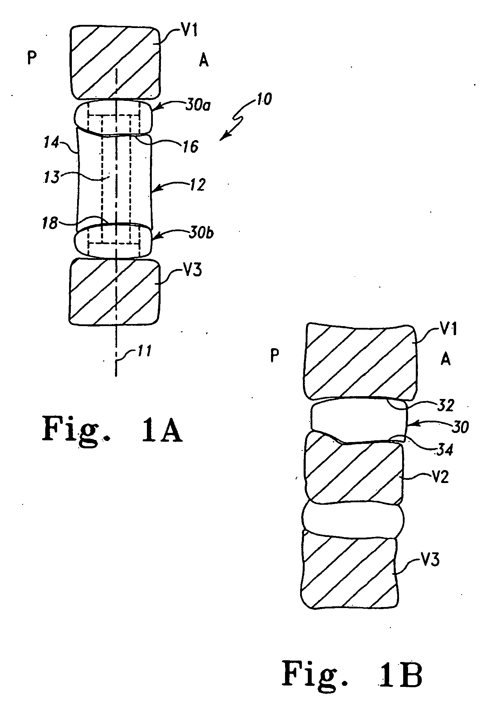

In FIG. 1A there is shown an elevation view of a vertebral replacement device 10 between vertebrae V1 and V3. One or more vertebrae, such as vertebrae V2 (Fig. 1B), have been removed in a corpectomy procedure. Vertebral replacement device 10 is positioned in the space between two remaining vertebrae V 1, V3 to provide support and stabilization of the spinal column. Vertebral replacement device 10 includes a connecting member 12, an upper member 30a, and a lower member 30b. Upper member 30a is positioned so that its superior surface contacts the lower endplate of vertebra V1, and lower member 30b is positioned so that its inferior surface contacts the upper endplate of vertebra V3. In Fig. 1B, upper member 30a is shown positioned in a disc space between adjacent vertebrae V1 and V2. Upper member 30a includes a superior surface 32 that contacts the lower endplate of vertebra V 1 and an inferior surface 34 that contacts the upper endplate of vertebra V2. In the illustrated embodiment, superior surface 32 includes a convexly curved profile while inferior surface 34 includes a planar profile. Lower member 30b could also be employed alone in a spinal disc space in lieu of or in addition to upper member 30a. As used herein, reference to the upper and lower members 30a, 30b is made collectively and individually as disc replacement member 30 or member 30.

Vertebral replacement device 10 is illustrated with a tubular form that extends along a longitudinal axis 11 and defines a chamber 13 extending therethrough along axis 11. Bone growth can occur through this chamber for fusion between the vertebral bodies supported at each end of device 10. Connecting member 12 includes a body 14 extending between an upper end 16 and an opposite lower end 18. Upper member 30a is positioned with its inferior surface in abutting engagement with upper end 16, and lower member 30b is positioned with its superior surface in abutting engagement with lower end 18. Upper end 16 includes a planar arrangement to provide an intimate fit with the planar inferior surface profile of upper member 30. Lower end 18 includes a concavely curved arrangement to provide an intimate fit with the convexly curved superior surface profile of lower member 30b.

The wall of body 14 and the walls of upper and lower members 30 are shown solid. Other embodiments contemplate the walls of any one or combination of the members 12, 30 may include one or more apertures in communication with the interior chamber thereof. Various shapes for the apertures are also contemplated, including non-circular shapes such as a square, diamond, triangular, oval and/or rectangular shapes, circular shapes, and/or polygonal shapes. The wall of any one or combination of the members 12, 30 can also include a number of holes extending at least partially therethrough. The holes can be threaded or otherwise sized and/or configured for engagement with one or more insertion instruments (not shown.)

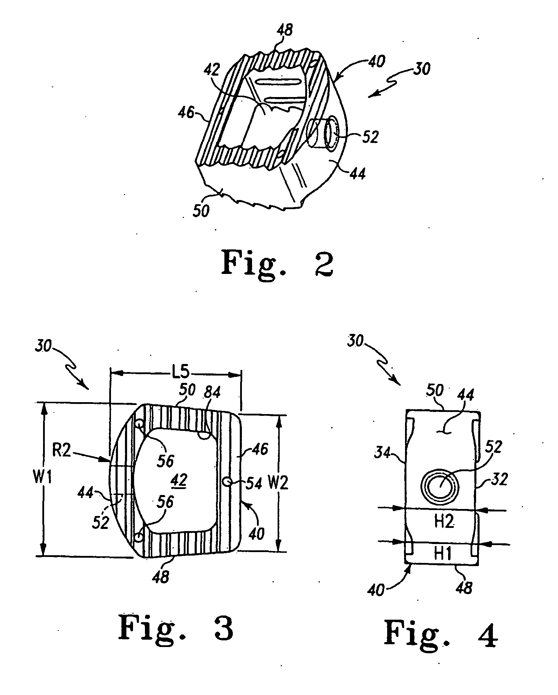

Referring further to Figs. 2-10, one embodiment of disc replacement member 30 will be discussed. Disc replacement member 30 includes an annular body 40 that extends around a central chamber 42. Body 40 includes an anterior wall 44 and an opposite posterior wall 46. A pair of opposite sidewalls 48, 50 extends between and connect anterior wall 44 to posterior wall 46. In the illustrated embodiment, the walls 44, 46, 48, 50 have a constant thickness between an outer surface thereof and an inner surface 84 thereof that defines the perimeter of chamber 42. Other embodiments contemplate that one or more of the walls has a thickness that varies from the other walls. In still other embodiments, the thickness along one or more of the walls can vary. In the illustrated embodiment, anterior wall 44 is convexly curved, and sidewalls 48, 50 converge from anterior wall 44 posterior to posterior wall 46. Posterior wall 46 extends linearly between sidewalls 48, 50.

Walls 44, 46, 48 and 50 define superior surface 32 of member 30 and inferior surface 34 of member 30. Superior and inferior surfaces 32, 34 provide bearing surface areas that contact the adjacent vertebral endplate when used as a disc replacement member or the adjacent endplate and the end of connecting member 12 when used in vertebral replacement device 10. Surfaces 32, 34 include a number of recesses extending thereacross to form elongated engaging members 36, 38 extending between sidewalls 48, 50 that are interrupted by central chamber 42 opening through superior and inferior surfaces 32, 34. Anterior wall 44 includes a hole 52 to receive an inserter instrument. Hole 52 can be threaded or in any other suitable configuration for engaging an inserter instrument.

In the illustrated embodiment, member 30 includes radiographic markers that are visible via a suitable surgical imaging system to determine the implanted location and orientation of body 40 without exposing the spinal column laterally. As shown in Fig. 3, body 40 includes superiorly and inferiorly extending markers 54, 56. Marker 54 is located in posterior wall 46 and provides an indication of the posterior location of body 40 when it is implanted and imaged. Markers 56 are located at opposite ends of anterior wall 44 adjacent respective ones of the sidewalls 48, 50. In a lateral image of the implanted member 30, markers 56 are aligned with one another in the anterior-posterior direction when body 40 is properly positioned in the space between vertebrae. After the implanted location and orientation of body 40 is determined by imaging, adjustments can be made in the positioning as deemed necessary and confirmed with additional images of the implanted body 40. Other embodiments contemplate body 40 is provided without markers and/or is made from a material that is viewable with surgical imaging systems without markers

Referring to Fig. 3, disc replacement member 30 includes an overall length L5 between the anterior-most side of anterior wall 44 and the posterior-most side of posterior wall 46. Anterior wall 44 includes an anterior surface that extends along a radius R2 between sidewalls 48, 50. Anterior wall 44 also defines an overall width W1 between sidewalls 48, 50, and posterior wall 46 defines an overall width W2 between sidewalls 48, 50. In one embodiment, the maximum width W1 in the medial lateral direction is greater than the length L5 in the anterior-posterior direction. Body 40 has a footprint sized to occupy more than one-half the area of the endplates of the vertebrae against which the bearing surfaces are positioned. In one specific embodiment, L5 is about 11 millimeters and radius R2 is about 11 millimeters. Width W1 is about 14 millimeters and width W2 is about 11 millimeters. Other embodiments contemplate other dimensions for length L5, radius R2 and widths W1, W2 so long as body 40 is sized in the anterior-posterior direction and medial-lateral direction for positioning in a spinal disc space.

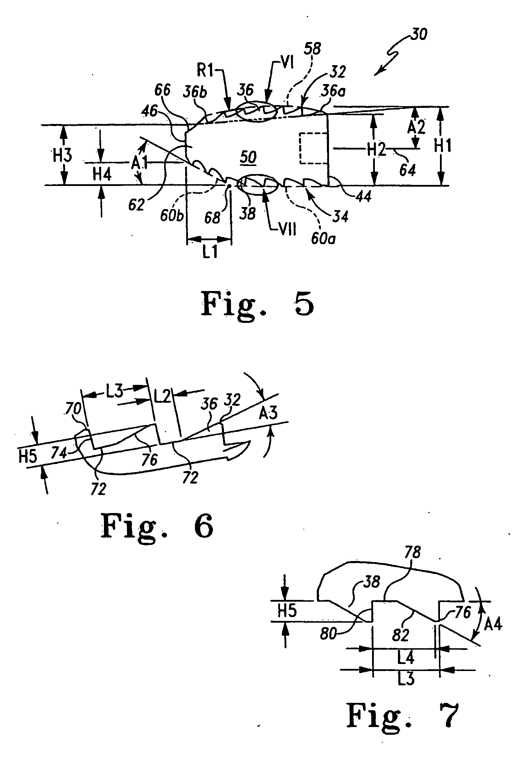

As shown in Fig. 5, one embodiment of disc replacement member 30 provides a specific configuration for superior and inferior surfaces 32, 34 that matches or closely corresponds to the profile of the respective adjacent vertebral endplate while minimizing endplate preparation. Superior surface 32 includes a convexly curved profile 58 between anterior and posterior walls 44, 46 while inferior surface 34 defines a planar profile with planar sections 60a, 60b between anterior and posterior walls 44, 46. First planar section 60a extends substantially orthogonally to anterior wall 44 while second planar section 60b is angled relative to first section 60a toward superior surface 32 to form a reduced height at posterior wall 46 with the curved superior surface profile 58. In one embodiment, convex profile 58 extends along a radius R1, and angled section 60b tapers at an angle A1 toward superior surface 32. In one specific embodiment, radius R1 is 17 millimeters and angle A1 is 30 degrees so that the planar sections 60a, 60b form an included angle of about 150 degrees between anterior wall 44 and posterior wall 46. Other embodiments contemplate other radii for R1 and taper angles for A1.

In the illustrated embodiment, superior surface 32 and inferior surface 34 are generally linear in the direction between sidewalls 48, 50 so that opposite sides of body 40 have the same height when viewed in any section parallel to posterior wall 46. The height of body 40 tapers generally toward posterior wall 46 to provide a wedge-shaped profile in the anterior-posterior direction. Planar section 60a of inferior surface 34 extends along a substantial portion of the length L5. In the illustrated embodiment, planar section 60a extends along more than half of the length L5. The convexly curved profile of superior surface 32 extends along substantially the entire length L5.

Body 40 defines an overall maximum height H1 between the outermost ends of engaging members 36, 38. Maximum height H1 ranges from about 5 millimeters to about 14 millimeters, although maximum heights less than 5 millimeters and greater than 14 millimeters are also contemplated based on the anatomy. Body 40 also includes an anterior end wall height H2 at the outer surface of anterior wall 44. Height H2 is about one millimeter less than height H1 since the anterior-most engaging member 36a includes a truncated outermost end in the illustrated embodiment. Anterior endwall height H2 also ranges from about 5 millimeters to about 14 millimeters, but can also be less than 5 millimeters or greater than 14 millimeters depending on the spinal anatomy. Body 40 also includes a posterior height H3 between superior location 66 and inferior location 68. Posterior nose 62 is formed by tapering surfaces located between locations 66, 68 and taper to the posterior-most side of posterior wall 46, as discussed further below. Height H3 corresponds to the height between superior and inferior surfaces 32, 34 at the locations 66, 68. Posterior height H3 is about 1.4 millimeters less than height H1 in one specific embodiment, and can range from about 4 millimeters to about 13 millimeters, but can also be less than 4 millimeters or greater than 13 millimeters depending on the spinal anatomy.

The taper location 68 for inferior surface 34 is located a distance L1 from the posterior-most side of nose 62. Distance L1 is about 3.5 millimeters in one embodiment. Taper angle A1 extends from this taper location 68 to the posterior-most surface of posterior wall 46. The location 66 adjacent superior surface 32 is at the location where superior taper angle A2 intersects a superior extension of the posterior-most surface of posterior wall 46. Taper angle A2 extends from the intersection of anterior-most engaging member 36a and the anterior-most surface of anterior wall 44 toward posterior wall 46. Taper angle A2 is measured from a central axis 64 that is parallel to planar section 60a of inferior surface 34 and orthogonal to the anterior-most surface of anterior wall 44. In one embodiment taper angle A2 is between about 4 to 5 degrees. Other embodiments contemplate other taper angles A2.

Nose 62 includes a height measured at the posterior-most surface of posterior wall 46. The nose height corresponds to posterior height H3 less the inferior taper height H4 of section 60b of inferior surface 34, and also less the superior taper height between superior location 66 and posterior-most surface of posterior wall 46. In one embodiment, inferior taper height H4 is about 2 millimeters, and the superior taper height is formed by a superior taper surface extends at an angle A1 from the posterior-most engaging member 36b the posterior-most side of nose 62. Accordingly, nose 62 is formed by superior and inferior surfaces that converge posteriorly at angle A1 to the posterior-most surface of posterior wall 46.

Referring now Fig. 6, additional details regarding engaging members 36 will be provided. Engaging members 36 are formed by a series of recesses in superior surface 32. The outermost ends 70 of engaging members 36 define surfaces that lie along an arc defined by radius R1, while the base 72 of engaging members 36 also lie along an arc that parallels the arc defined by radius R1. Trailing end walls 74 extend generally orthogonally to the respective adjacent base 72, while leading end walls 76 are sloped along angle A3 relative to base 72. The base 72 has a length L2 along the body 40, and trailing end walls 74 are separated by length L3. Engaging members 36 have a height H5 extending from base 72 to outermost end 70. In one embodiment, length L2 is about 0.5 millimeters, and length L3 is about 1.5 millimeters. Height H5 is about 0.5 millimeters, and angle A3 is about 16 degrees. Other embodiments contemplate other dimensions and/or configurations for engaging members 36.

Referring now Fig. 7, additional details regarding engaging members 38 will be provided. Engaging members 38 are formed by a series of recesses formed in inferior surface 34, and are similar to engaging members 36 except that the outermost ends 76 and the bases 78 are planar to lie in planar section 60a or planar section 60b. Trailing end walls 80 extend generally orthogonally to the respective adjacent base 78, while leading end walls 82 are sloped along angle A4 relative to base 78. The base 78 has a length L2 along the body 40, and trailing end walls 80 are separated by length L3. Outermost ends 76 define a planar surface having a length corresponding to the difference between length L3 and the length L4 between the trailing end wall 80 and the end of the sloped portion of leading end wall 82. Engaging members 38 have a height H5 extending from base 78 to outermost end 76. In one embodiment, length L3 is about 1.5 millimeters and length L4 is about 1.375 millimeters. Height H5 is about 0.5 millimeters, and angle A4 is about 30 degrees. Other embodiments contemplate other dimensions and/or configurations for engaging members 38.

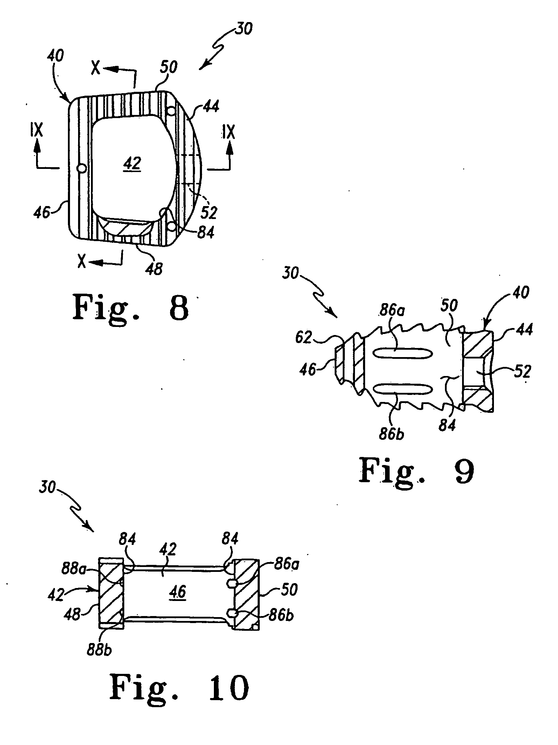

Figs. 8-10 are provided to show, among other features, receptacles of disc replacement member 30 that positively engage it to connecting member 12. In Fig. 8 a plan view of disc replacement member 30 is shown in partial section along inner wall surface 84 that surrounds chamber 42. As shown in Fig. 9, sidewall 50 includes a superior receptacle 86a and an inferior receptacle 86b. Sidewall 48 similarly includes superior receptacle 88a and inferior receptacle 88b as shown in Fig. 10. Receptacles 86a, 86b and receptacles 88a, 88b extend parallel to one another and are elongated to extend in the anterior-posterior direction along inner surface 84. Superior receptacles 86a, 88a are located to engage connecting member 12 when superior surface 32 is positioned in abutting engagement with connecting member 12. Inferior receptacles 86b, 88b are located to engage connecting member 12 when inferior surface 34 is positioned in abutting engagement with connecting member 12. As shown in Fig. 10, receptacles 86a, 86b and receptacles 88a, 88b are defined by shallow, V-shaped recesses in inner wall surface 84 that do not extend through the respective sidewall 48, 50. The V-shaped recesses facilitate attachment and detachment of the disc replacement member 30 from connecting member 12 while providing sufficient axial restraint to prevent detachment unless sufficient force is applied to overcome the attachment arrangement. Other embodiments contemplate other shapes for the receptacles, including circular, oval, triangular, non-circular, and irregular shapes. The receptacles also extend completely through the wall of member 30 in another embodiment.

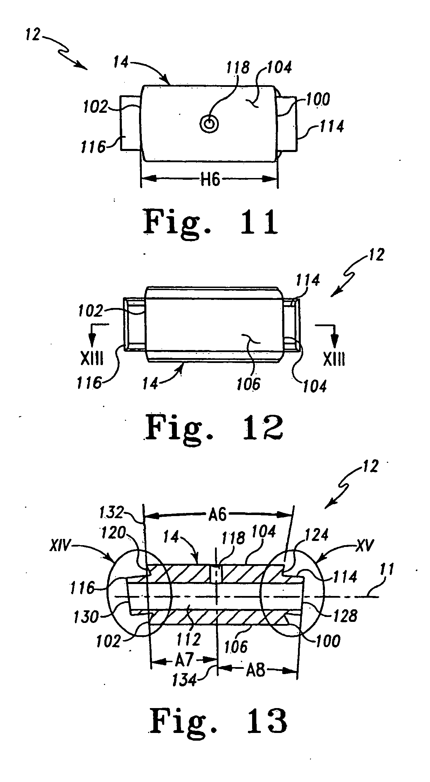

Figs. 11-17 show one embodiment for connecting member 12. Connecting member 12 includes body 14 extending between an upper or superior end 100 and a lower or inferior end 102. Body 14 includes a tubular form and an outer surface profile that defines a perimeter like that of disc replacement member 30. Body 14 includes an anterior wall 104 with a convexly curved outer surface, a posterior 106 with a linear outer surface, and sidewalls 108, 110 that have linear outer surfaces converging from anterior wall 104 toward posterior wall 106. Anterior wall 104 includes a hole 118 for engagement by an insertion tool. Hole 118 can be configured like hole 52 discussed above or include any suitable configuration.

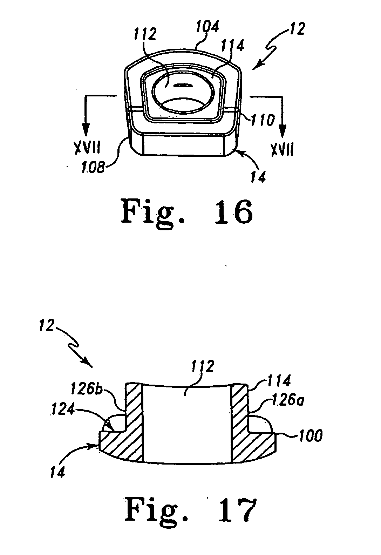

Connecting member 12 further includes an upper extension 114 extending from upper end 100 and a lower extension 116 extending from lower end 102. Upper and lower extensions 114, 116 include an outer perimeter that matches the shape of interior chamber 42 so that inner wall surface 84 resides in close or intimate relationship with the respective upper and lower extensions 114, 116. Connecting member 12 further defines an axially extending central chamber 112 that extends through upper and lower ends 100, 102 and also through upper and lower extensions 114, 116. In the illustrated embodiment, chamber 112 includes an oval shape that is elongated in the direction between sidewalls 108, 110 as shown in Fig. 16. Other embodiments contemplate other shapes for chamber 112, including circular, non-circular, polygonal, and irregular shapes. In still other embodiments, chamber 112 includes one or more partitions dividing it into chamber portions. In yet another embodiment, chamber 112 is not continuous in the axial direction, but includes one or more ledges, walls, or partitions along the length of body 14 extending therein.

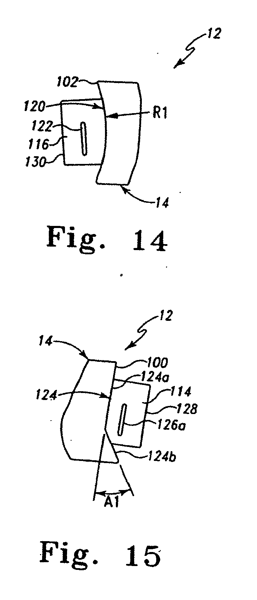

As shown in Fig. 14, lower end 102 includes an end bearing surface 120 that has a concave shape defined by radius R1. Bearing surface 120 conforms to the shape of superior surface 32 of disc replacement member 30 so that the outermost ends of engaging members 36 are fully supported on bearing surface 120. Lower extension 116 includes at least one engaging member 122 extending outwardly therefrom that is engageable in one or more of the adjacent superior receptacles 86a, 88a of the disc replacement member 30 placed on the inferior end of connecting member 12. Engaging members 122 can be provided on both sides of lower extension 116 that are sized and shaped to be removably received in the receptacles 86a, 88a so that member 30 is removably attachable to connecting member 12 at lower end 102.

As shown in Fig. 15, upper end 100 includes an end bearing surface 124 with first planar section 124a and second planar section 124b. Second planar section 124b is angled relative to first planar section 124a at angle A1. Planar sections 124a, 124b match the configuration of planar sections 60a, 60b of inferior surface 34 of disc replacement member 30 so that the outermost ends of engaging members 38 are fully supported along bearing surface 124. Upper extension 114 includes engaging members 126a, 126b (Fig. 17) extending outwardly therefrom on opposite sides thereof that are engageable in the adjacent inferior receptacle 86b, 88b of the disc replacement member 30 placed in the superior end 100 of connecting member 12. Engaging members 126a, 126b are sized and shaped to be removably received in the respective receptacles 86b, 88b so that disc replacement member 30 is removably attachable to connecting member 12 at upper end 100.

Connecting member 12 is employed with disc replacement members 30 attached at each end thereof for vertebral body replacement procedures. Bearing surfaces 120, 124 extend completely around the respective extensions 114, 116 to provide bearing support of the disc replacement members 30. Connecting member 12 can be provided with various heights between upper and lower ends depending on the number and size of the vertebrae to be replaced. Heights H6 ranging from about 5 millimeters to about 62 millimeters or more are contemplated. In one form, a set of connecting members 12 of various heights are provided in a kit along with a set of disc replacement members 30 of various heights. The connecting member and disc replacement members providing the desired overall height are selected and assembled to form a vertebral replacement device 10.

Furthermore, when device 10 is employed in vertebral body replacement procedures, the endplates of the vertebrae to be supported may not extend parallel to one another but rather are angled to diverge from another in the anterior direction. Connecting member 12 is provided with various angular orientations between upper and lower ends 100, 102 and the outer ends 128, 130 of the upper and lower extensions 114, 116. For example, upper and lower ends 100, 102 can be formed with an angle A6 between planar section 124a and the axis 132 extending between the anterior and posterior sides of bearing surface 120 at lower end 102. Angle A7 is defined between axis 132 and a central axis 134 that extends orthogonally to the central longitudinal axis 11. Angle A8 is defined between axis 134 and outer end 128. Outer end 130 and axis 134 also define an angle A8 therebetween.

In one application, the angulation at the ends of connecting member 12 provides an attachment arrangement to permit superior and inferior ends of the disc replacement devices 30 attached to the respective ends of connecting member 12 to converge posteriorly (diverge anteriorly) to better fit with the endplates of the vertebrae remaining to be supported. The shorter the span between vertebrae, the less angulation is provided between the disc replacement members 30. In one embodiment, where height H6 is small and about 5 millimeters, angle A6 is between about 1 and 2 degrees, angle A7 is between about 1 to 2 degrees, and angle A8 is about 3 degrees. In another embodiment where height H6 is large and about 62 millimeters, angle A6 is between about 19 to 20 degrees, angle A7 is between about 7 to 8 degrees, and angle A8 is about 12 degrees. Other lengths contemplate other angular arrangements between ends 128, 130 and other portions of the connecting body 12 to provide the desired fit with the spinal column anatomy. For example, ends 128, 130 extend parallel to one another in one embodiment. In another embodiment, ends 128, 130 are angled to converge anteriorly and diverge posteriorly.

Vertebral replacement device 10 can be used to replace a vertebra that has been removed from the spinal column segment using known techniques. Device 10 is assembled by securing upper member 30a to upper end 100 of connecting member 12 and securing lower member 30b to the lower end 102 of connecting member 12. This provides a vertebral replacement device 10 that has an overall anterior height that is equal to the sum of the height H6 of body 14 and the height of members heights H2 upper and lower members 30a, 30b.

Although not required, it is contemplated that height H6 is representative of that of the removed vertebra or vertebrae and heights H2 are representative of the heights of the respective disc spaces between the removed vertebrae and the remaining vertebrae V1, V3. Although not shown, it is also contemplate that a stabilization construct may be engaged to and extending between vertebrae V1, V3 or vertebrae V1, V2 along the exterior surfaces thereof outside the space between the vertebrae to provide additional support and stabilize the spinal column segment before, during and, if the construct is non-resorbable and left in the patient, after fusion. The stabilization construct can be a rod system, plate system or artificial ligament system. It is further contemplated that stabilization construct could be attached to any portion of the vertebrae, including the anterior, antero-lateral, lateral, postero-lateral or posterior portions.

It is also contemplated that connecting member 12 could be provided with one end configured to bear against a vertebral endplate, and that only one of the upper and lower members 30a, 30b is engaged to the other end of connecting member 12. The assembled device could then be placed between adjacent vertebrae with an end of connecting member 12 and an end of the selected upper or lower member 30a, 30b in contact with the adjacent vertebral endplates.

While the invention has been illustrated and described in detail in the drawings and foregoing description, the same is to be considered as illustrative and not restrictive in character.

## Claims

1. A spinal interbody fusion member (30), comprising:
a body (40) including an anterior side (44), a posterior side (46), and opposite lateral sides (48, 50) extending between the anterior and posterior sides (44, 46), wherein the body (40) defines a superior bearing surface (32) and an opposite inferior bearing surface (34),
wherein the superior bearing surface (32) defines a convexly curved profile (58) between the anterior and posterior sides (44, 46) extending along substantially the entire length (L5) of the body in the anterior-posterior direction,
wherein the inferior bearing surface (34) defines a planar profile between the anterior and posterior sides (44, 46) including a first planar section (60a) extending from the anterior side (44) and a second planar section (60b) extending between the first planar section (60a) and a leading end nose (62) at the posterior side (46), and
wherein the first planar section (60a) extends substantially orthogonally to the anterior side (44),
**characterised in that**
the second planar section (60b) is angled relative to the first planar section (60a) toward the superior bearing surface (32) to form a reduced height at the posterior side (46) with the curved superior surface profile (58).

2. The member (30) of claim 1, wherein the body (40) includes a chamber (42) opening through the superior and inferior bearing surfaces (32, 34).

3. The member (30) of claim 1, wherein the body (40) includes engaging members (36, 38) across at least a portion of the superior and inferior bearing surfaces (32, 34).

4. The member (30) of claim 1, wherein the inferior and superior bearing surfaces (32, 34) taper toward the leading end nose (62) at the posterior side (46).

5. The member (30) of claim 1, wherein the body (40) includes a height (H1, H2, H3) between the superior and inferior surfaces (32, 34) sized to replace at least one vertebrae (V1, V2, V3) of a spinal column.

6. The member (30) of claim 1, wherein the body (40) includes a height (H1, H2, H3) between the superior and inferior surfaces (32, 34) sized for positioning in a disc space between adjacent vertebrae (V1, V2; V2, V3).

7. The member (30) of claim 1, wherein the body (40) includes a width (W1) between the opposite lateral sides (48, 50), wherein the width (W1) is greater than the length (L5).

8. The member (30) of claim 1, wherein the opposite lateral sides (48, 50) converge toward one another toward the posterior side (46).

9. The member (30) of claim 1, wherein the anterior side (44) includes a convexly curved outer surface extending between the lateral sides (48, 50).

10. The member (30) of claim 2, wherein an inner surface (84) extending around the chamber (42) is defined interior of each of the anterior side (44), the posterior side (46), and the opposite lateral sides (48, 50), the inner surface (48) including at least one elongated receptacle (86a, 86b, 88a, 88b) extending therein along at least one of the opposite lateral sides (48, 50).

11. The member (30) of claim 10, wherein the at least one receptacle (86a, 86b, 88a, 88b) includes a receptacle (86a, 86b, 88a, 88b) in the inner surface (84) along each of the opposite lateral sides (48, 50).

12. The member (30) of claim 3, wherein one or more of the engaging members (36, 38) include outer-most end surfaces (70, 76) along respective ones of the superior and inferior bearing surfaces (32, 34), one or more of the engaging members (36, 38) including a trailing end wall (74, 80) oriented toward the anterior side (44) and a leading end wall (76, 82) oriented toward the posterior side (46), wherein the trailing end wall (74, 80) is generally orthogonally oriented to the end bearing surface (70, 76) and the leading end wall (76, 82) is obliquely oriented to the respective end surface (70, 76).

13. The member (30) of claim 1, wherein the first planar section (60a) is orthogonally oriented to an anterior surface of the anterior side (32) and the first and second planar sections (60a, 60b) form an angle of about 150 degrees between the leading end nose (62) and the anterior surface.

14. The member (30) of claim 1, wherein the first planar section (60a) of the inferior bearing surface (34) extends along more than half the length (L5) of the inferior surface (34) between the anterior and posterior sides.

## Patentansprüche

1. Ein spinal-Zwischenkörper-Fusions-Element (30), aufweisend:
einen Körper (40), umfassend eine anteriore Seite (44), eine posteriore Seite (46) und gegenüberliegende laterale Seiten (48, 50), welche sich zwischen der anterioren und der posterioren Seite (44, 46) erstrecken, wobei der Körper (40) eine superiore Trag- bzw. Auflagefläche (32) und eine gegenüberliegende inferiore Trag- bzw. Auflagefläche (34) definiert,
wobei die superiore Auflagefläche (32) zwischen der anterioren und der posterioren Seite (44, 46) ein konvex gekrümmtes Profil (58) definiert, welches sich entlang im Wesentlichen der gesamten Länge (L5) des Körpers in der anterior-posterior-Richtung erstreckt,
wobei die inferiore Auflagefläche (34) zwischen der anterioren und der posterioren Seite (44, 46) ein planares Profil definiert, umfassend einen ersten planaren Abschnitt (60a), der sich von der anterioren Seite (44) aus erstreckt, sowie einen zweiten planaren Abschnitt (60b), welcher sich zwischen dem ersten planaren Abschnitt (60a) und einer führenden Endnase (62) an der posterioren Seite (46) erstreckt, und
wobei der erste planare Abschnitt (60a) sich im Wesentlichen orthogonal zu der anterioren Seite (44) erstreckt,
**dadurch gekennzeichnet, dass**
der zweite planare Abschnitt (60b) relativ zu dem ersten planaren Abschnitt (60a) abgewinkelt ist bzw. einen Winkel bildet zu der superioren Auflagefläche (32) hin, um mit dem gekrümmten superioren Flächenprofil (58) an der posterioren Seite (46) eine reduzierte Höhe zu formen.

2. Das Element (30) nach Anspruch 1, wobei der Körper (40) eine Kammer (42) aufweist, welche durch die superiore und die inferiore Auflagefläche (32, 34) hindurch offen ist.

3. Das Element (30) nach Anspruch 1, wobei der Körper (40) Eingriff-Elemente (36, 38) über zumindest einen Abschnitt der superioren und der inferioren Auflagefläche (32, 34) aufweist.

4. Das Element (30) nach Anspruch 1, wobei die inferiore und die superiore Auflagefläche (32, 34) sich zu der führenden Endnase (62) an der posterioren Seite (46) hin verjüngen.

5. Das Element (30) nach Anspruch 1, wobei der Körper (40) zwischen der superioren und der inferioren Fläche (32, 34) eine Höhe (H1, H2, H3) aufweist, welche bemessen ist, um zumindest einen Wirbel (V1, V2, V3) von einer Wirbelsäule zu ersetzen.

6. Das Element (30) nach Anspruch 1, wobei der Körper (40) zwischen der superioren und der inferioren Fläche (32, 34) eine Höhe (H1, H2, H3) aufweist, welche bemessen ist zum Positionieren in einen Scheiben-Raum zwischen benachbarten Wirbeln (V1, V2; V2, V3).

7. Das Element (30) nach Anspruch 1, wobei der Körper (40) zwischen den gegenüberliegenden lateralen Seiten (48, 50) eine Breite (W1) aufweist, wobei die Breite (W1) größer ist als die Länge (L5).

8. Das Element (30) nach Anspruch 1, wobei die gegenüberliegenden lateralen Seiten (48, 50) zu der posterioren Seite (46) hin zueinander konvergieren.

9. Das Element (30) nach Anspruch 1, wobei die anteriore Seite (44) eine konvex gekrümmte Außenfläche hat, welche sich zwischen den lateralen Seiten (48, 50) erstreckt.

10. Das Element (30) nach Anspruch 2, wobei eine Innenfläche (84), welche sich um die Kammer (42) erstreckt, innerhalb von jeder von der anterioren Seite (44), der posterioren Seite (46) und den gegenüberliegenden lateralen Seiten (48, 50) definiert ist, wobei die Innenfläche (48) zumindest eine langgestreckte Aufnahme (86a, 86b, 88a, 88b) aufweist, welche sich darin entlang zumindest einer der gegenüberliegenden lateralen Seiten (48, 50) erstreckt.

11. Das Element (30) nach Anspruch 10, wobei die zumindest eine Aufnahme (86a, 86b, 88a, 88b) eine Aufnahme (86a, 86b, 88a, 88b) in der Innenfläche (84) entlang jeder der gegenüberliegenden lateralen Seiten (48, 50) aufweist.

12. Das Element (30) nach Anspruch 3, wobei ein oder mehrere der Eingriff-Elemente (36, 38) äußerste Endflächen (70, 76) aufweisen, entlang der superioren bzw. der inferioren Auflagefläche (32, 34), ein oder mehrere der Eingriff-Elemente (36, 38) eine zurückhängende bzw. nachlaufende Endwand (74, 80), welche zu der anterioren Seite (44) hin ausgerichtet ist, und eine führende Endwand (76, 82) aufweisen, welche zu der posterioren Seite (46) ausgerichtet ist, wobei die nachlaufende Endwand (74, 80) im Allgemeinen orthogonal ausgerichtet ist zu der End-Auflage-Fläche (70, 76) und die führende Endwand (76, 82) schräg zu der jeweiligen Endfläche (70, 76) ausgerichtet ist.

13. Das Element (30) nach Anspruch 1, wobei der erste planare Abschnitt (60a) orthogonal ausgerichtet ist zu einer anterioren Fläche der anterioren Seite (32) und der erste und der zweite planare Abschnitt (60a, 60b) einen Winkel von ungefähr 150 Grad zwischen der führenden Endnase (62) und der anterioren Fläche formen.

14. Das Element (30) nach Anspruch 1, wobei der erste planare Abschnitt (60a) der inferioren Auflagefläche (34) sich entlang mehr als die Hälfte der Länge (L5) der inferioren Fläche (34) zwischen der anterioren und der posterioren Seite erstreckt.

## Revendications

1. Elément d'arthrodèse vertébrale intersomatique (30) comprenant :
un corps (40) comportant un côté antérieur (44), un côté postérieur (46) et des côtés latéraux opposés (48, 50) s'étendant entre les côtés antérieur et postérieur (44, 46), dans lequel le corps (40) définit une surface d'appui supérieure (32) et une surface d'appui inférieure (34) opposée,
dans lequel la surface d'appui supérieure (32) définit un profil incurvé de manière convexe (58) entre les côtés antérieur et postérieur (44, 46) s'étendant sensiblement le long de toute la longueur (L5) du corps dans la direction antéropostérieure,
dans lequel la surface d'appui inférieure (34) définit un profil plan entre les côtés antérieur et postérieur (44, 46) comprenant une première section plane (60a) s'étendant à partir du côté antérieur (44) et une seconde section plane (60b) s'étendant entre la première section plane (60a) et un nez d'extrémité d'attaque (62) au niveau du côté postérieur (46), et
dans lequel la première section plane (60a) s'étend sensiblement de manière orthogonale par rapport au côté antérieur (44), **caractérisé en ce que** la seconde section plane (60b) est coudée par rapport à la première section plane (60a) vers la surface d'appui supérieure (32), afin de former une hauteur réduite au niveau du côté postérieur (46) avec le profil de surface supérieure incurvé (58).

2. Elément (30) selon la revendication 1, dans lequel le corps (40) comprend une chambre (42) s'ouvrant à travers les surfaces d'appui supérieure et inférieure (32, 34).

3. Elément (30) selon la revendication 1, dans lequel le corps (40) comprend des éléments de mise en prise (36, 38) sur au moins une partie des surfaces d'appui supérieure et inférieure (32, 34).

4. Elément (30) selon la revendication 1, dans lequel les surfaces d'appui inférieure et supérieure (32, 34) se rétrécissent progressivement vers le nez d'extrémité d'attaque (62) au niveau du côté postérieur (46).

5. Elément (30) selon la revendication 1, dans lequel le corps (40) comprend une hauteur (H1, H2, H3) entre les surfaces supérieure et inférieure (32, 34), dimensionnées pour remplacer au moins une vertèbre (V1, V2, V3) d'une colonne vertébrale.

6. Elément (30) selon la revendication 1, dans lequel le corps (40) comprend une hauteur (H1, H2, H3) entre les surfaces supérieure et inférieure (32, 34), dimensionnées pour le positionnement dans un espace de disque entre les vertèbres (V1, V2 ; V2, V3) adjacentes.

7. Elément (30) selon la revendication 1, dans lequel le corps (40) comprend une largeur (W1) entre les côtés latéraux opposés (48, 50), la largeur (W1) étant supérieure à la longueur(L5).

8. Elément (30) selon la revendication 1, dans lequel les côtés latéraux opposés (48, 50) convergent l'un vers l'autre vers le côté postérieur (46).

9. Elément (30) selon la revendication 1, dans lequel le côté antérieur (44) comprend une surface externe incurvée de manière convexe s'étendant entre les côtés latéraux (48, 50).

10. Elément (30) selon la revendication 2, dans lequel une surface interne (84) s'étendant autour de la chambre (42) est définie à l'intérieur de chacun parmi le côté antérieur (44), le côté postérieur (46) et les côtés latéraux opposés (48, 50), la surface interne (48) comprenant au moins un réceptacle allongé (86a, 86b, 88a, 88b) s'étendant à l'intérieur de cette dernière le long d'au moins l'un des côtés latéraux opposés (48, 50).

11. Elément (30) selon la revendication 10, dans lequel le au moins un réceptacle (86a, 86b, 88a, 88b) comprend un réceptacle (86a, 86b, 88a, 88b) dans la surface interne (84) le long de chacun des côtés latéraux opposés (48, 50).

12. Elément (30) selon la revendication 3, dans lequel un ou plusieurs des éléments de mise en prise (36, 38) comprend des surfaces d'extrémité situées le plus à l'extérieur (70, 76) le long des surfaces respectives des surfaces d'appui supérieure et inférieure (32, 34), un ou plusieurs des éléments de mise en prise (36, 38) comprenant une paroi d'extrémité de fuite (74, 80) orientée vers le côté antérieur (44) et une paroi d'extrémité d'attaque (76, 82) orientée vers le côté postérieur (46), dans lequel la paroi d'extrémité de fuite (74, 80) est généralement orientée de manière orthogonale par rapport à la surface d'appui d'extrémité (70, 76), et la paroi d'extrémité d'attaque (76, 82) est orientée de manière oblique par rapport à la surface d'extrémité (70, 76) respective.

13. Elément (30) selon la revendication 1, dans lequel la première section plane (60a) est orientée de manière orthogonale par rapport à une surface antérieure du côté antérieur (32), et les première et seconde surfaces planes (60a, 60b) forment un angle d'environ 150 degrés entre le nez d'extrémité d'attaque (62) et la surface antérieure.

14. Elément (30) selon la revendication 1, dans lequel la première section plane (60a) de la surface d'appui inférieure (34) s'étend le long de plus de la moitié de la longueur (L5) de la surface inférieure (34) entre les côtés antérieur et postérieur.
